# EUROPEAN PATENT SPECIFICATION

(11) **EP 0 857 049 B1**
(45) Date of publication and mention of the grant of the patent: **04.06.2003**
(21) Application number: 96928249.0
(22) Date of filing: 20.08.1996
(51) Int. Cl.: A61F 13/472, A61F 13/15

(54) **ELASTICATED INCONTINENCE PRODUCT WITH IMPROVED FLUID HANDLING CAPACITY**
ELASTISCHES INKONTINENZ-PRODUKT MIT VERBESSERTER FLUSSIGKEITS-AUFNAHME KAPAZITÄT
ARTICLE ELASTIQUE POUR INCONTINENCE AVEC MEILLEURE CAPACITE D'ABSORPTION DES LIQUIDES

(30) Priority: 23.08.1995 IT TO950697
(43) Date of publication of application: 12.08.1998
(73) Proprietor: THE PROCTER & GAMBLE COMPANY, Cincinnati, Ohio 45202 (US)
(72) Inventor: PALUMBO, Gianfranco, D-61352 Bad Homburg (DE)
(74) Representative: McGregor, Judit Ester
(86) International application number: US9613449
(87) International publication number: WO97007763

(56) References cited:
- EP-A- 0 386 816
- EP-A- 0 426 197
- WO-A-95/00093
- US-A- 4 425 130
- US-A- 4 892 536
- US-A- 4 935 021
- US-A- 5 171 236
- US-A- 5 197 959
- US-A- 5 269 775
- US-A- 5 295 988
- US-A- 5 324 278
- US-A- 5 411 498
- US-A- 5 558 656

## Description

This invention relates to an absorbent article. It is particularly concerned with an absorbent article, for example in the form of a pad, which can be used by women suffering from light and moderate incontinence. The invention will be so described below. However, the invention is of more general applicability in relation to the absorption of body fluids, either urine or menstrua.

One problem associated with such articles is that urine may not go directly into the article, and be absorbed therein, but may flow along the user's body in a rearward direction and escape without ever entering the absorbent material of the article. This is particularly true in the case of gushes of urine which are of low volume or low flow rate.

EP-A-0426197 describes a sanitary napkin with a wick means and with an upwardly facing body-contacting portion. US5171236 describes a diaper with a void space formed by the presence of a spacer. US5269775 and EP-A-0386816 describe articles with an elastically extendible topsheet, the articles of the former document also having a void space. US4892536 describes diapers with a void space and with elastic strands in the topsheet. US5295988 describes folded sanitary napkins with elastic elements.

In the accompanying drawings:
Figure 1 is a perspective view of an embodiment of the article described in the said copending application;
Figure 2 is a longitudinal section through the article of Figure 1, taken along the longitudinal centre line thereof;
Figure 3 is a transverse section through the article of Figure 1, taken on the transverse centre line thereof; and
Figure 4 is a view similar to Figure 3 but showing a modified form of the article.

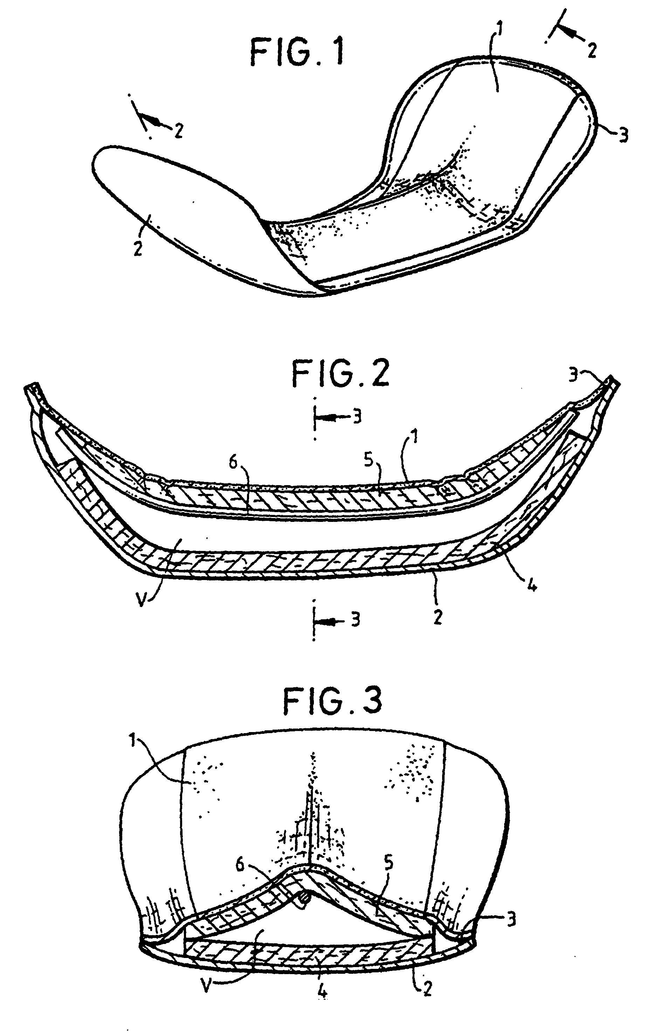

Figures 1 to 3 show an absorbent sanitary pad for use by a woman suffering from light incontinence. It comprises a fluid-permeable topsheet 1, a fluid-impermeable backsheet 2 which is sealed thereto, by a seal 3 at the periphery of the article, for example by heat sealing or adhesive. The article further comprises an absorbent core 4, for example one which contains an absorbent gelling material (AGM), and a secondary topsheet 5 which serves to receive fluid and transfer it to the core.

The materials used for the topsheet, backsheet and absorbent core may be those which are conventionally used for absorbent sanitary products. However, attention is directed to International Patent Publication WO94/28838 which describes an absorbent product having a secondary topsheet of high bulkiness, and to WO94/01069 and PCT/EP94/04215 which describe cores including AGM's, for further details of some suitable materials.

The article further comprises an elastication system which serves to cause the article to curve in a way which enables it to conform closely to the user's body and at least substantially prevent, or reduce, the risk of urine escaping past it. In Figures 1 to 3 this elastication system comprises an elastic thread 6 which runs along at least a major portion of the length of the article, preferably substantially the whole length of the article, beneath the secondary topsheet 5, and is attached at its ends, or along its whole length, or at selected points along its length, to the secondary topsheet and/or to the core. As can be seen, this causes the pad to assume a configuration which is upwardly concave as seen in longitudinal section (Figure 2), and upwardly convex as seen in transverse section (Figure 3). The pad is thus resiliently raised towards the body of the user and held in close contact with it, so providing a better fit and a better ability to acquire even the lightest release of urine. The core is relatively stiff, and stays substantially flat as view in transverse section, thus enabling it to maintain good contact with the user's panty.

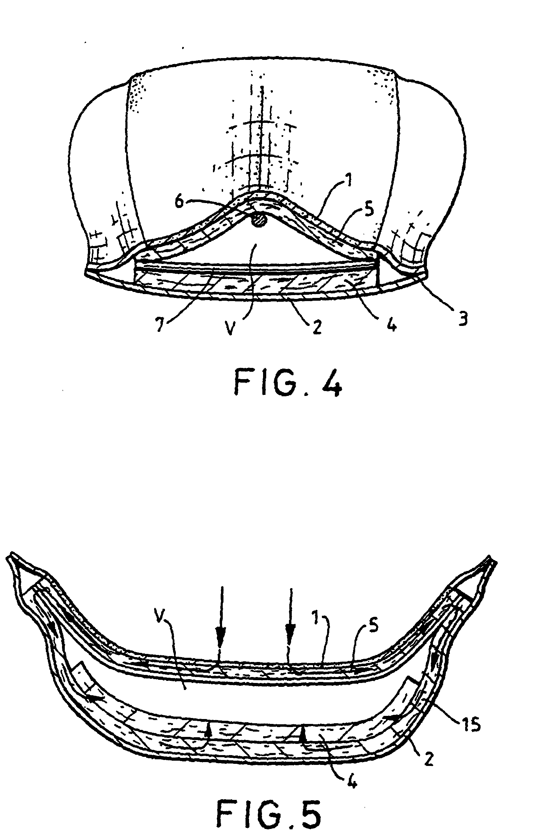

Figure 4 shows a modified article which comprises in addition an elastic thread 7 which runs transversely above the core 2, and below the secondary topsheet 5 with its longitudinal elastic thread 6. The transverse elastic thread 7 is shown fixed to the secondary topsheet 5, though if it were slightly longer it could alternatively, or additionally, be fixed to the primary topsheet 1. In any event, it is preferably fixed only at its ends. By pulling the sides of the secondary topsheet and/or primary topsheet inwards, the transverse elastic thread assists in giving the article, as seen in transverse section, the desired upwardly convex shape.

Various other modifications are also possible. For example, a transverse elastic thread may be provided, with a longitudinal elastic thread, or a plurality of transverse threads could be provided (with a longitudinal thread), the plurality being arranged close to one another or spaced along the length of the article, and the single longitudinal thread may be replaced by a plurality of longitudinal threads arranged close to one another.

The articles described above with reference to Figures 1 to 4, particularly when the or each elastic thread is positioned beneath the secondary topsheet, are advantageous in terms of their fit to the user and, hence, their ability to collect even small quantities of urine. However, the construction employed means that a void space, indicated as V in Figures 2, 3, and 4, is formed between the secondary topsheet and the core. The purpose of the secondary topsheet is to receive fluid temporarily and transmit it to the core for storage, and this void space may cause difficulties as regards the transmission process.

It is an object of the present invention to provide an article of the type described above with reference to Figures 1 to 4, but in which this fluid transmission problem is eliminated or at least reduced.

According to the invention there is provided an absorbent sanitary article, which comprises a fluid-absorbing core, a first fluid-receiving layer positioned on one side of the core and spaced therefrom over at least part of its surface to define a void between the core and the said fluid-receiving layer, and a second fluid-receiving layer positioned on the opposite side of the core to the first fluid-receiving layer, the said second fluid-receiving layer being in fluid communication with the core and with the first and fluid-receiving layer.

Two embodiments of the invention are shown in Figures 5 to 7 of the accompanying drawings, in which:
Figure 5 is a longitudinal section, taken on the longitudinal centre line of the first embodiment of the article;

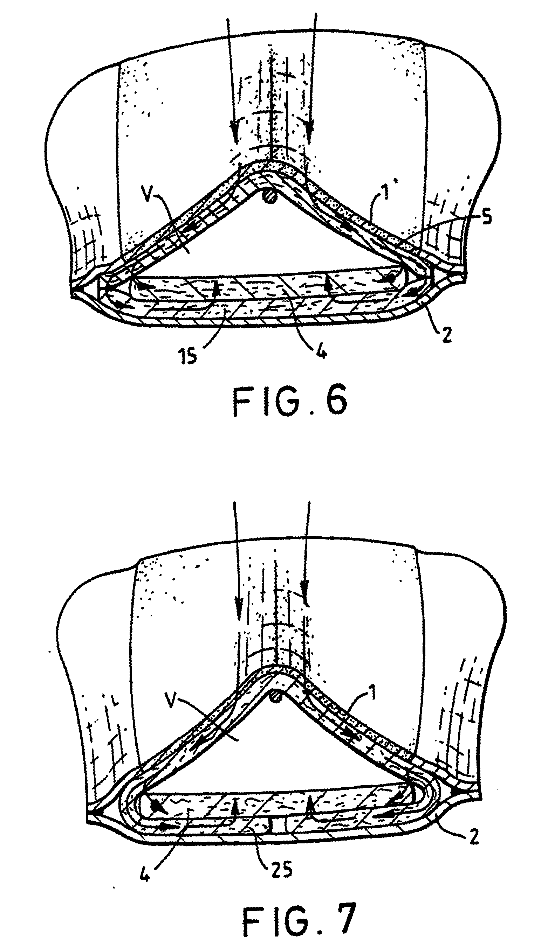

Figure 6 is a transverse section, taken on the transverse centre line of the embodiment of Figure 5; and
Figure 7 is a view similar to Figure 6, but showing a second embodiment.

Figures 5 and 6 show a light incontinence pad having, like the article of Figures 1 to 3, a topsheet 1, a backsheet 2, a core 4 and a secondary, fluid-receiving, topsheet 5. In addition, there is a further fluid-receiving sheet 15 which is positioned between the core and the backsheet 2. The sheets 5 and 15 are in fluid communication with one another over at least part of the edge regions thereof. As shown in Figures 5 and 6 respectively, the sheets are both longer and wider than the core, and are bonded to one another, for example by adhesive, at their longitudinal ends and lateral edges, the seal between them extending completely around the periphery of both sheets. However, it is alternatively possible, for example, for the sheets to be bonded to one another only at their longitudinal ends (in which case it is not essential that they should be wider than the core), or only at their lateral edges, in which case it is not essential that they should be longer than the core.

Figures 5 and 6 show by means of arrows the flow path of fluid which enters the article through the liquid-permeable topsheet 1. As can be seen, it flows longitudinally and laterally through the secondary sheet 5 and thence into the sheet 15, where again it flows longitudinally and laterally. The fluid can then be absorbed from the sheet 15 by the core 4, which is in intimate contact with it.

Under some circumstances, for example in the case of a sudden large gush of liquid, some of the liquid acquired by the secondary topsheet 5 may enter the void V directly from the secondary topsheet, and such liquid can then be absorbed by the core 4 without passing through the sheet 15.

The sheet 15 may be of the same material as the sheet 5, though it need not be. Whether or not the two sheets are of the same material, they may have the same or different thicknesses. Attention is directed to our Italian Patent Application No. TO94A000982, filed 1st December 1994, which describes an absorbent pad having fluid-receiving layers both above and below the core. Some suitable materials for the purpose are described therein.

In the modification shown in Figure 7, the sheets 5 and 15 are replaced by a single sheet 25 which surrounds the core 4 and void V and is bonded to itself along its lateral edges to give it a tubular form. It may also, or alternatively, be bonded to itself along its end edges.

In a further modification, a transversely extending elastic thread is provided, which runs across the surface of the core facing the void V and is attached at its ends to the secondary topsheet 5 and or the topsheet 1, as is shown in Figure 4 in relation to an article without a second fluid-receiving sheet 15.

## Claims

1. An absorbent sanitary pad, which comprises a fluid-absorbing core (4), a first fluid-receiving layer (1) positioned on one side of the core and spaced therefrom over at least part of its surface to define a void (V) between the core and the said fluid-receiving layer, and a second fluid-receiving layer positioned on the opposite side of the core to the first fluid-receiving layer (5), the said second fluid-receiving layer being In fluid communication with the core and with the first and fluid-receiving layer, the pad comprising elastication means located between the core and the first fluid-receiving layer, the elastication means being in tension when the article is in a generally flat condition and acting to lift the first fluid-receiving layer away from the fluid-absorbing layer to create the said void, **characterised in that** said elastication means comprises at least one elastic thread (6) running substantially along at least a major portion of the longitudinal centre line of the pad, beneath the second fluid receiving layer, and **in that** the pad is upwardly concave as seen in longitudinal section and upwardly convex as seen in transverse section.

2. A pad according to claim 1, wherein the or each said thread (6) is attached at its ends to at least one of the first fluid-receiving layer (1) and the core (4).

3. A pad according to claim 1, wherein the or each said thread (6) is attached at selected points along its length to at least one of the first fluid-receiving layer (1) and the core (4).

4. A pad according to any preceding claim, wherein the first and second fluid-receiving layers (1 and 5) are bonded to one another along a first edge region and along a second edge region at the opposite side or end of the layers.

5. A pad according to any one of claims 1 to 3, wherein the first and second fluid-receiving layers (1 and 5) are bonded to one another at least substantially along their complete peripheries.

6. A pad according to any one of claim 1 to 3, wherein the first and second fluid-receiving layers (1 and 5) are constituted by a single sheet of material folded around the core (4).

7. A pad according to any preceding claim, further comprising a fluid-impermeable backsheet (2) on the side of the second fluid-receiving layer (5) remote from the core (4).

## Patentansprüche

1. Absorbierendes Hygiene-Kissen, welches aufweist einen Flüssigkeits-absorbierenden Kern (4), eine erste Flüssigkeits-aufnehmende Lage (1), die auf einer Seite des Kerns angeordnet ist und zu diesem über mindestens einen Teil seiner Oberfläche beabstandet ist, um einen Hohlraum (V) zwischen dem Kern und der Flüssigkeits-aufnehmenden Lage zu bilden, und eine zweite Flüssigkeits-aufnehmende Lage, die auf der der ersten Flüssigkeits-aufnehmenden Lage (5) gegenüberliegenden Seite des Kerns angeordnet ist, wobei die zweite Flüssigkeits-aufnehmende Lage in Flüssigkeits-Verbindung mit dem Kern und mit der ersten Flüssigkeits-aufnehmenden Lage steht, wobei das Kissen ein zwischen dem Kern und der ersten Flüssigkeits-aufnehmenden Lage angeordnetes Elastifizierungs-Mittel umfasst, wobei das Elastifizierungs-Mittel gespannt ist, wenn sich das Kissen in einem im Wesentlichen flachen Zustand befindet, und wirkt, um die erste Flüssigkeits-aufnehmende Lage von der Flüssigkeits-absorbierenden Lage wegzuziehen, um den Hohlraum zu bilden, **dadurch gekennzeichnet, dass** das Elastifizierungs-Mittel mindestens einen elastischen Faden (6) aufweist, der im Wesentlichen entlang mindestens eines Haupt-Abschnitts der Längs-Mittellinie des Kissens unter der zweiten Flüssigkeits-aufnehmenden Lage verläuft, und dass das Kissen nach oben konkav ist, betrachtet im Längsschnitt, und nach oben konvex ist, betrachtet im Querschnitt.

2. Kissen nach Anspruch 1, wobei der oder jeder Faden (6) an seinen Enden an der ersten Flüssigkeits-aufnehmenden Lage ( 1 ) und/oder dem Kern (4) angebracht ist.

3. Kissen nach Anspruch 1, wobei der oder jeder Faden (6) an ausgewählten Punkten über seine Länge an der ersten Flüssigkeits-aufnehmenden Lage ( 1 ) und/oder dem Kern (4) angebracht ist.

4. Kissen nach einem der vorherigen Ansprüche, wobei die erste und zweite Flüssigkeits-aufnehmende Lage (1 und 5) über einen ersten Rand-Bereich und über einen zweiten Rand-Bereich an der gegenüberliegenden Seite oder dem gegenüberliegenden Ende der Lagen miteinander verbunden sind.

5. Kissen nach einem der Ansprüche 1 bis 3, wobei die erste und zweite Flüssigkeits-aufnehmende Lage 1 und 5 mindestens im Wesentlichen entlang ihrer vollständigen Peripherien miteinander verbunden sind.

6. Kissen nach einem der Ansprüche 1 bis 3, wobei die erste und zweite Flüssigkeits-aufnehmende Lage (1 und 5) durch eine einzelne Schicht des um den Kern (4) gefalteten Materials gebildet sind.

7. Kissen nach einem der vorherigen Ansprüche, ferner umfassend eine flüssigkeitsundurchlässige Außenlage (2) auf der Seite der zweiten Flüssigkeits-aufnehmenden Lage (5), die entfernt von dem Kern (4) ist.

## Revendications

1. Tampon hygiénique absorbant qui comprend une âme d'absorption de fluide (4), une première couche de réception de fluide (1) placée sur un côté de l'âme et espacée de celle-ci sur au moins une partie de sa surface afin de définir un vide (V) entre l'âme et ladite couche de réception de fluide, et une deuxième couche de réception de fluide placée sur le côté opposé de l'âme par rapport à la première couche de réception de fluide (5); ladite deuxième couche de réception de fluide étant en communication de fluide avec l'âme et avec la première couche de réception de fluide, le tampon comprenant un moyen d'élastification situé entre l'âme et la première couche de réception de fluide, le moyen d'élastification étant tendu lorsque l'article est dans un état globalement plat et agissant de manière à soulever la première couche de réception de fluide à distance de la couche d'absorption de fluide afin d'engendrer ledit vide, **caractérisé en ce que** ledit moyen d'élastification comprend au moins un fil élastique (6) dont le tracé suit sensiblement au moins une partie principale de la ligne médiane longitudinale du tampon, sous la deuxième couche de réception de fluide, et **en ce que** le tampon est concave vers le haut lorsqu'on l'observe en coupe longitudinale, et convexe vers le haut lorsqu'on l'observe en coupe transversale.

2. Tampon selon la revendication 1, dans lequel ledit fil ou chacun desdits fils (6) est fixé au niveau de ses extrémités au moins, soit à la première couche de réception de fluide (1), soit à l'âme (4).

3. Tampon selon la revendication 1, dans lequel ledit fil ou chacun desdits fils (6) est fixé en des points sélectionnés suivant sa longueur au moins, soit à la première couche de réception de fluide (1), soit à l'âme (4).

4. Tampon selon l'une quelconque des revendications précédentes, dans lequel les première et deuxième couches de réception de fluide (1 et 5) sont reliées l'une à l'autre le long d'une première région de bord et le long d'une deuxième région de bord au niveau du côté ou de l'extrémité opposé(e) des couches.

5. Tampon selon l'une quelconque des revendications 1 à 3, dans lequel les première et deuxième couches de réception de fluide (1 et 5) sont reliées l'une à l'autre au moins sensiblement le long de leurs périphéries complètes.

6. Tampon selon l'une quelconque des revendications 1 à 3, dans lequel les première et deuxième couches de réception de fluide (1 et 5) sont constituées par une feuille unique de matériau pliée autour de l'âme (4).

7. Tampon selon l'une quelconque des revendications précédentes, comprenant, en outre, une feuille de fond (2) imperméable aux fluides sur le côté de la deuxième couche de réception de fluide (5) à distance de l'âme (4).
